# EUROPEAN PATENT SPECIFICATION

(11) **EP 1 184 351 B1**
(45) Date of publication and mention of the grant of the patent: **12.11.2003**
(21) Application number: 00119048.7
(22) Date of filing: 02.09.2000
(51) Int. Cl.: C03C 27/08, A61N 1/375

(54) **Method for brazing two glass components**
Verfahren zum Hartlöten zweier Glaskomponenten
Procédé de brasage de deux pièces en verre

(43) Date of publication of application: 06.03.2002
(73) Proprietor: Medos SA, 2400 Le Locle (CH)
(72) Inventor: Tardy, Yanik, 2206 Genevey/Coffrane (CH); Ginggen, Alec, 3225 Muentschemier (CH); Steinmann, Pierre-Albert, 2325 Les Planchettes (CH); Meuterlos, Stéphane, 25500 Morteau (FR)
(74) Representative: Micheli & Cie

(56) References cited:
- US-A- 5 866 851
- CAMERON T ET AL: "MICROMODULAR IMPLANTS TO PROVIDE ELECTRICAL STIMULATION OF PARALYZED MUSCLES AND LIMBS" IEEE TRANSACTIONS ON BIOMEDICAL ENGINEERING,US,IEEE INC. NEW YORK, vol. 44, no. 9, 1 September 1997 (1997-09-01), pages 781-790, XP000696675 ISSN: 0018-9294
- ZIAIE B ET AL: "A HERMETIC GLASS-SILICON MICROPACKAGE WITH HIGH-DENSITY ON-CHIP FEEDTHROUGHS FOR SENSORS AND ACTUATORS" JOURNAL OF MICROELECTROMECHANICAL SYSTEMS,US,IEEE INC. NEW YORK, vol. 5, no. 3, 1 September 1996 (1996-09-01), pages 166-178, XP000636779 ISSN: 1057-7157
- ZIAIE B ET AL: "A SINGLE-CHANNEL IMPLANTABLE MICROSTIMULATOR FOR FUNCTIONAL NEUROMUSCULAR STIMULATION" IEEE TRANSACTIONS ON BIOMEDICAL ENGINEERING,US,IEEE INC. NEW YORK, vol. 44, no. 10, 1 October 1997 (1997-10-01), pages 909-920, XP000703528 ISSN: 0018-9294
- DATABASE COMPENDEX [Online] ENGINEERING INFORMATION, INC., NEW YORK, NY, US; ANONYMOUS: "Nerve implant could provide hope for paralysis patients" Database accession no. EIX97433802791 XP002162417 & BIOMED INSTRUM TECHNOL;BIOMEDICAL INSTRUMENTATION & TECHNOLOGY JUL-AUG 1997 ASSOC FOR THE ADVANCEMENT OF MEDICAL INSTRUMENTATION, ARLINGTON, VA, USA, vol. 31, no. 4, July 1997 (1997-07), pages 399-400,

## Description

This invention relates to a method of brazing two glass components at low temperature (i.e. under glass melting temperature) in view of producing a leak tight container for encapsulating electronic components. More particularly the method of the present invention may be applied to manufacture a glass encapsulated pressure transducer, which can then be implanted in a human body.

Another object of the present invention relates to the encapsulating container obtained with the manufacturing method.

A further object of the invention relates to an encapsulated implantable pressure transducer according to the teaching of the manufacturing method.

The method for brazing to glass components will be described in relation with the manufacturing of an implantable pressure transducer but may of course be applied to any other fields requiring low temperature leak tight brazing of two glass components so as to manufacture a glass encapsulated implantable device.

Many medical applications require implantable measurement devices; for example, intra cranial pressure is a key parameter for regulating the flow rate in a shunt of a shunted hydrocephalic patient. Once implanted, the pressure transducer may be inductively coupled to a reading unit which energises the transducer and allows the transmission of data corresponding to the measured pressure between the transducer and the reading unit.

Such implantable systems or devices like pacemakers for example already exist on the market and are generally active components having their own source of energy. They are encapsulated in a titanium container which is usually hermetically sealed thanks to a laser bonding.

These existing devices are difficult to manufacture and the use of a titanium container is usually not suitable for passive transducers, which have to be energised by inductive coupling thanks to an external reading unit.

The choice of encapsulating electronic components in a glass container has many advantages over the prior art. Firstly the type of the glass may be chosen with an expansion coefficient matching the one of the silicon and therefore no differential expansion occurs between the container and the electronic parts when the sealing operation is performed. Glass is also a good barrier to liquid and thus forms a leak tight container once sealed. Furthermore the glass may be considered as chemically inert and bio compatible and therefore suitable for long term implantation in a human body. It is also to be noted that glass is permeable to RF waves which permits the use of passive telemetry once the device is implanted in a human body. Glass is also transparent to x-ray which may be an advantage or a requirement for medical implanted devices.

In order not to damage the electronic components encapsulated in the glass container the brazing should be made at low temperature, preferably less than 200 degrees Celsius. The resulting container should also be leak tight and have suitable mechanical resistance to stress.

The aim of the present invention is to solve these problems by providing a method of brazing two glass components at low temperature in order to form a suitable container for encapsulating electronic components.

This goal is achieved by a method having the characteristic recited in claim 1. The resulting hermetically sealed glass container is the object of claim 11.

Further features and other objects and advantages of this invention will become clear from the following detailed description made with reference to the accompanying drawings illustrating in a schematic and non-limiting way one embodiment of an encapsulated pressure transducer.
Fig. 1 is an exploded perspective view of an encapsulated pressure transducer.
Fig. 2 is a perspective view of the encapsulated pressure transducer illustrated at fig 1, once the container is sealed.
Fig. 3 is perspective exploded view of an alternate embodiment of the base plate of the encapsulated pressure transducer.

The method for brazing two glass components at low temperature will now be described in details. The objective is to braze two pieces made of a borosilicate glass for example of the Pyrex type with a low temperature solder so that the resulting bond is leak tight (bubble free and uniformly distributed) and strong enough for the intended applications. This is achieved by first depositing a first thin metallic layer on at least one portion of the borosilicate glass surface. The thickness of this layer is comprised between 50 and 150 nanometers, preferably 100 nanometers (nm). The first metallic layer may be chosen between any metallic element provided that it presents a good adherence to glass. Titanium and chrome are example of materials that may be deposited on glass as a first layer. This first metallic layer may be deposited by know techniques like physical vapor deposition (PVD) or any other method that allows the deposition of a metallic layer on a glass substrate. Next, a second metallic layer suitable for performing brazing operation is deposited above the first metallic layer, this layer having a thickness comprised between 2 and 10 microns (µm), preferably 5 microns (µm). The second metallic layer may be chosen between any metal which does not become oxidized. Different metals like gold (Au), silver (Ag), nickel (Ni) or stainless steel may be used for example to form this second metallic layer. The above mentioned operations are performed on the corresponding borosilicate glass part to which the first part has to be brazed. An optional third metallic layer may be deposited on the second layer, this third layer having the same properties as the second layer. For cost reasons the preferred method uses only two metallic layers but the results would be identical if more than two layers were deposited.

The next step consist of positioning a low temperature solder element in between the two second layers of the respective borosilicate glass parts and to heat the assembly in a furnace so that the brazing occurs.

Any low temperature solder may be used provided that this solder element is compatible with the second metallic layer, presents a good absorptivity and is bubble free. InAg, AgPbSn, SnBi, SnZn or SnInZn amongst other are example of solder which may be used as a solder element for brazing the two glass components.

The preferred solder material are low temperature solders such as ln97Ag3 or Sn77.2In20Ag2.8 which melting point are respectively 143°C and 187°C. The optimal solder thickness is between 50 and 100 microns (µm).

The assembly is then heated in an oven at a temperature of about 200°C for a time comprised between 1 and 15 minutes, preferably 5 minutes.

For medical applications, like the manufacturing of glass encapsulated devices, the use of a SnInAg braze is preferred because the vapor sterilization temperature (often 132°C) is very close to the melting point of InAg.

As an alternative, the solder element may be deposited above the second layer either on one element or on both elements to be brazed.

Tests have shown that the optimal results were obtained with a first titanium layer, a second gold layer and a SnInAg solder (thickness 50-100µm) and heating during 5 minutes at a temperature of 200°C in an oven under helium atmosphere. In the resulting assembly, the braze was uniformly distributed and bubble free and therefore perfectly leak free. The bonding between the two borosilicate glass parts also presented good mechanical characteristics under strains.

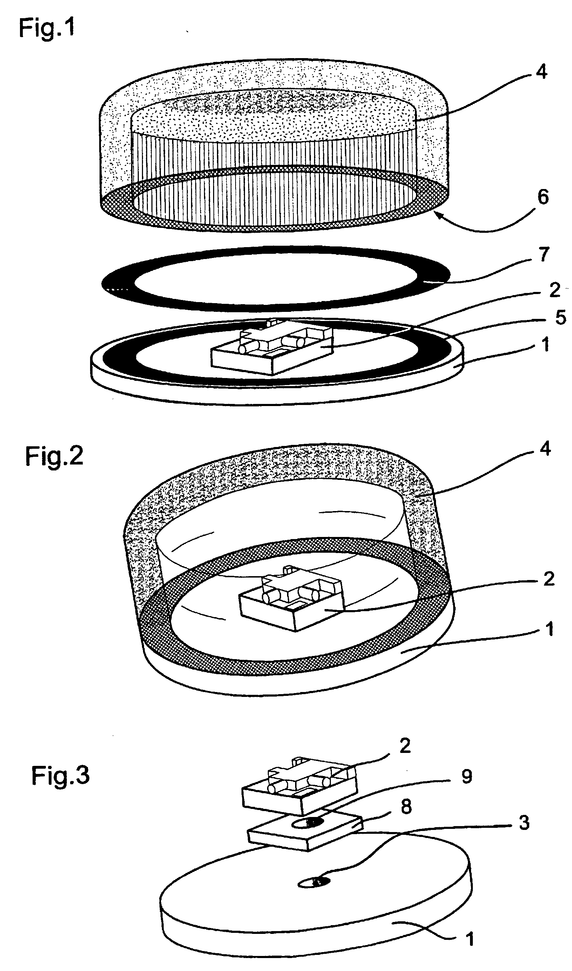

Now referring to figure 1, there is shown a container for encapsulating a pressure sensor. For clarity purpose in the accompanying drawings, only the pressure transducer is shown and not the other electronic components which may be installed anywhere inside the glass container. The container comprises a circular base plate 1 made of a borosilicate glass such as Pyrex on which is bonded a pressure transducer 2. The bonding of the pressure transducer on the base plate 1 may be achieved by known techniques like anodic bonding. These known techniques permit the bonding of a silicon component like a pressure transducer over a glass surface and will not be described in detail in the present application. The base plate 1 comprises a hole 3 (see fig. 3) and the pressure transducer is bonded on the base plate 1 so that the membrane of the pressure transducer 2 is located in front of the hole 3. The container further comprises a closed cylindrical cover 4 having radial dimension corresponding to the base plate 1 so as to form a container or capsule once sealed over the base plate.

The base plate 1 comprises a bi-metallic layer 5 on at least one portion of its surface. This bi-metallic layer 5 forms, in the example shown, a thin ring located on an outer diameter of the base plate 1. The bi-metallic layer 5 is composed of a first layer of titanium on which a second layer of gold has been deposited. The thickness of the two layers are those mentioned in the above description.

The bottom edge 6 of the cover 4 also comprises a portion of its surface, forming a ring on which a bi metallic layer of the same composition is deposited. For sealing the container, thus encapsulating the pressure transducer 2, a low temperature solder braze ring 7, for example made of SnInAg, is inserted between the base plate 1 and the cover 4, the cover 4 is then urged against the base plate 1 so as to close the container.

This assembly, shown at fig. 2 is then heated for 5 minutes in an oven under helium atmosphere so as to perform the brazing between the two bi-metallic surfaces located on the base plate 1 and at the bottom of the cover 4. The resulting container contains the pressure transducer 2 and optional other electronic components in a leak tight glass container.

Fig 3. shows an alternate embodiment of the encapsulated device in which the pressure transducer 2 is not directly bonded to the base plate 1 but to a glass support 8. The support 8 may be made of a borosilicate glass such as Pyrex for example. In that embodiment, the pressure transducer 2 may be bonded to the Pyrex support 8 at the wafer level (before cutting the wafer) by conventional anodic bonding. The support 8 and the base plate 1 are then brazed together according to the above disclosed method. To that extent a first metallic layer like titanium for example is deposited on at least a portion of the upper face of the base plate 1 and to the corresponding bottom surface of the support 8. Then a second metallic layer for example of gold is deposited on the first metallic layers. A low temperature brazing element having shape and dimension corresponding to the above mentioned bi-metallic layers is then sandwiched between the base plate 1 and the support 8. The brazing is done when the assembly is heated and allows the bonding of both the cover 4 on the base plate 1 and the support 8 on the base plate 1. In that embodiment, the glass support 8 comprises a central hole 9 corresponding to the central hole 3 of the base plate 1. The pressure transducer 2 is first bonded on the glass support 8 so that the membrane of the pressure transducer 2 is positioned above the central hole 9. Then the support 8 is positioned so that the hole 9 in the support 8 and the hole 3 in the base plate 1 coincides on at least one part of their opened surface. In the resulting encapsulated pressure transducer the liquid may interact on the membrane of the pressure transducer through the hole of the base plate 1 and the hole of the glass support 8.

In the above description, reference was made to the manufacturing of an implantable pressure transducer, but many other implantable devices (micro pump, flow sensor, chemical sensor) may be encapsulated in a leak tight glass container using the same method of manufacturing and the scope of the claimed invention is not restricted to the above disclosed pressure transducer.

The shape and dimensions of the base plate 1 and the corresponding cover 4 may obviously also be different than the cylindrical example shown on fig 2. Depending on the intended application, the container may for example be shaped as a cube, a sphere or any other volume made of two corresponding parts that can be linked together to form a closed container.

This encapsulation method in a glass container is particularly well suited for manufacturing inductively coupled implantable medical devices. The use of an inexpensive material is an advantage and the containers are easier and cheaper to manufacture than existing titanium containers.

## Claims

1. A method of brazing two borosilicate glass pieces in view of obtaining a leak tight bonding between the two glass pieces comprising the steps of:
- depositing a first metallic layer on at least one portion of the surface of each glass pieces that should be brazed together
- depositing at least a second metallic layer on the above mentioned first deposited metallic layer
- inserting a low temperature solder element between the opposite bi-metallic layer surfaces of the glass pieces
- urging the two glass pieces together and heating the assembly at a temperature allowing the brazing of the solder element, said temperature being less than the glass melting point.

2. Method according to claim 1 **characterized in that** the solder element is deposited on the second layer of at least one the glass piece to be brazed

3. Method according to one of the preceding claims **characterized in that** the thickness of the first metallic layer is comprised between 50 and 150 nanometers, preferably 100 nanometers (nm) and **in that** the thickness of the second metallic layer is comprised between 2 and 10 microns (µm), preferably 5 microns (µm)

4. Method according to one of the preceding claims, **characterized in that** the fist metallic layer is made of titanium, the second metallic layer of gold and **in that** the low temperature solder element is made of SnInAg in the following proportions Sn 77.2% In 20& Ag 2.8%.

5. Method according to one of the preceding claims **characterized in that** the heating is performed in an oven under helium atmosphere at a temperature around 200°C for a period comprised between 1 and 15 minutes, preferably 5 minutes.

6. Method of encapsulating electronic components in a glass container made of at least two corresponding elements that can be linked together to form a closed container **characterized in that** it comprises the following steps:
- depositing a first metallic layer on at least a portion of the first element
- depositing at least a second metallic layer on top of the first mentioned metallic layer
- depositing the first and the second above mentioned layers on the corresponding surface of the second element forming the container
- arranging the electronic components inside the container
- inserting a low temperature solder element in between the two corresponding elements of the container
- heating the assembly.

7. Method of encapsulation according to claim 6, **characterized in that** the container comprises a base plate and a corresponding cylindrical cover made of a borosilicate glass and that the first deposited metallic layer is of titanium, the second layer of gold and that the solder is made of SnInAg.

8. Implantable medical device **characterized in that** the electronic components thereof are encapsulated in a glass container comprising two pieces that are brazed together according to the method of claim 1 so as to form a leak tight container.

9. Implantable pressure sensor **characterized in that** it comprises a pressure transducer bonded to a borosilicate glass base plate having a hole on a portion of its surface and **in that** a borosilicate glass cover is brazed at low temperature on the base plate according to the method recited in one of the claims 1 to 5 so as to leak tight seal the cover on the base plate.

10. Implantable pressure sensor according to claim 9, **characterized in that** the pressure transducer is bonded on a borosilicate glass support and **in that** the support is brazed according the method of claim 1 on the base plate of a borosilicate glass container comprising a base plate and a corresponding cover.

11. Glass container for encapsulating electronic components in view of being implanted in a human or animal body, **characterized in that** it comprises a first element and a corresponding cover element brazed together according to the method of claim 1.

## Patentansprüche

1. Verfahren zum Hartlöten zweier Stücke von Borosilicatglass mit dem Ziel, eine dichte Verkittung zwischen den beiden Glasstücken zu erhalten, die Schritte umfassend:
- eine erste metallische Schicht auf zumindest einem Abschnitt der Oberfläche jedes der zusammenzulötenden Glasstücke abzuscheiden,
- zumindest eine zweite metallische Schicht auf der oben erwähnten, ersten abgeschiedenen metallischen Schicht abzuscheiden,
- ein niedrigschmelzendes Lotelement zwischen die sich gegenüberstehenden bimetallischen Schichtoberflächen der Glasstücke einzufügen,
- die beiden Glasstücke zusammenzudrücken und das Ganze auf eine Temperatur zu erhitzen, bei der ein Hartlöten des Lotelements erfolgen kann, wobei diese Temperatur niedriger als der Schmelzpunkt des Glases ist.

2. Verfahren nach Anspruch 1, **dadurch gekennzeichnet, dass** das Lotelement auf der zweiten Schicht von zumindest einem der hartzulötenden Glasstücke abgeschieden wird.

3. Verfahren nach einem der vorangehenden Ansprüche, **dadurch gekennzeichnet, dass** die Dicke der ersten metallischen Schicht zwischen 50 und 150 Nanometern und bevorzugt bei 100 Nanometern (nm) liegt und dass die Dicke der zweiten metallischen Schicht zwischen 2 und 10 Mikrometern (ìm) und bevorzugt bei 5 Mikrometern (ìm) liegt.

4. Verfahren nach einem der vorangehenden Ansprüche, **dadurch gekennzeichnet, dass** die erste metallische Schicht aus Titan, die zweite metallische Schicht aus Gold hergestellt ist und das niedrigschmelzende Lotelement aus SnInAg in den folgenden Verhältnissen: Sn 77,2 %, In 20 % und Ag 2,8 %, hergestellt ist.

5. Verfahren nach einem der vorangehenden Ansprüche, **dadurch gekennzeichnet, dass** das Heizen in einem Ofen in Heliumatmosphäre bei einer Temperatur um 200 °C über eine Zeitdauer erfolgt, die zwischen 1 und 15 Minuten und bevorzugt bei 5 Minuten liegt.

6. Verfahren zum Verkapseln elektronischer Komponenten in einem Glasbehälter, der aus zumindest zwei entsprechenden Elementen hergestellt ist, die zu einem geschlossenen Behälter zusammengefügt werden können, **dadurch gekennzeichnet, dass** es die folgenden Schritte umfasst:
- eine erste metallische Schicht auf zumindest einem Abschnitt des ersten Elements abzuscheiden,
- zumindest eine zweite metallische Schicht auf der erwähnten ersten metallischen Schicht abzuscheiden,
- die oben erwähnte erste und zweite Schicht auf der entsprechenden Oberfläche des zweiten, den Behälter bildenden Elements abzuscheiden,
- ein niedrigschmelzendes Lotelement zwischen die zwei entsprechenden Elemente des Behälters einzufügen,
- das Ganze zu erhitzen.

7. Verfahren zum Verkapseln nach Anspruch 6, **dadurch gekennzeichnet, dass** der Behälter eine Grundplatte und einen entsprechenden zylindrischen Deckel aus Borosilicatglas umfasst und dass die erste abgeschiedene metallische Schicht aus Titan, die zweite Schicht aus Gold und das Lot aus SnInAg hergestellt ist.

8. Implantierbare medizinische Vorrichtung, **dadurch gekennzeichnet, dass** ihre elektronischen Komponenten in einem Glasbehälter verkapselt sind, der aus zwei Stücken besteht, die nach dem Verfahren des Anspruchs 1 zusammengelötet sind, um einen dichten Behälter zu bilden.

9. Implantierbarer Druckfühler, **dadurch gekennzeichnet, dass** er einen Druckumsetzer umfasst, der auf eine Borosilicatglas-Grundplatte aufgekittet ist, die ein Loch auf einem Abschnitt ihrer Oberfläche hat, und dadurch, dass ein Borosilicatglas-Deckel bei niedriger Temperatur nach dem in einem der Ansprüche 1 bis 5 angeführten Verfahren auf die Grundplatte aufgelötet ist, um den Deckel dicht auf die Grundplatte aufzuschmelzen.

10. Implantierbarer Druckfühler nach Anspruch 9, **dadurch gekennzeichnet, dass** der Druckumformer auf einen Borosilicatglas-Träger aufgekittet ist und dass der Träger nach dem Verfahren des Anspruchs 1 auf die Grundplatte eines Borosilicatglas-Behälters aufgelötet ist, der eine Grundplatte und einen entsprechenden Deckel umfasst.

11. Glasbehälter zum Verkapseln elektronischer Komponenten mit dem Ziel, in einen Menschen- oder Tierkörper implantiert zu werden, **dadurch gekennzeichnet, dass** er ein erstes Element und ein entsprechendes Deckelelement umfasst, die nach dem Verfahren des Anspruchs 1 zusammengelötet sind.

## Revendications

1. Procédé pour souder deux pièces en verre borosilicate en vue d'obtenir une liaison étanche entre les deux pièces de verre comprenant les étapes suivantes :
- déposer une première couche métallique sur au moins une partie de la surface de chacune des pièces de verre devant être soudées ensemble,
- déposer au moins une seconde couche métallique par-dessus la première couche déposée,
- insérer un élément de soudure à basse température entre les deux couches bi-métalliques opposées des deux surfaces des pièces en verre,
- serrer les deux pièces de verre ensemble et chauffer l'ensemble à une température permettant la fusion de l'élément de soudure, cette température étant inférieure au point de fusion du verre.

2. Procédé selon la revendication 1, **caractérisé par le fait que** l'élément de soudure est déposé sur la seconde couche d'au moins une des deux pièces en verre à souder.

3. Procédé selon l'une des revendications précédentes, **caractérisé par le fait que** l'épaisseur de la première couche métallique est comprise entre 50 et 150 nanomètres (nm) de préférence 100 nanomètres (nm) et en ce que l'épaisseur de la seconde couche métallique est comprise entre 2 et 10 microns (µm), de préférence 5 microns (µm).

4. Procédé selon l'une des revendications précédentes, **caractérisé par le fait que** la première couche métallique est composée de titane, la seconde couche métallique est composée d'or et que l'élément de soudure à basse température est constitué d'un alliage de SnAgln dans les proportions suivantes : Sn : 27 % In : 20 % Ag : 2 %

5. Procédé selon l'une des revendications précédentes, **caractérisé par le fait que** l'opération de chauffage est réalisée dans un four sous atmosphère d'hélium à une température de 200°C environ et durant une période comprise entre 1 et 15 minutes, de préférence 5 minutes.

6. Procédé d'encapsulation de composants électroniques dans un conteneur en verre composé d'au moins deux éléments correspondants qui peuvent être assemblés ensemble de manière à former un conteneur fermé, **caractérisé par le fait qu'**il comporte des étapes suivantes :
- déposer une première couche métallique sur au moins une portion du premier élément,
- déposer une seconde couche métallique par-dessus la première couche métallique mentionnée ci-dessus,
- déposer la première et la seconde couche métallique référencée ci-dessus sur la surface correspondante du second élément formant le conteneur,
- arranger les composants électroniques à l'intérieur du conteneur,
- insérer un élément de soudure à basse température entre les deux parties correspondantes du conteneur,
- chauffer l'ensemble.

7. Procédé d'encapsulation selon la revendication 6, **caractérisé par le fait que** le conteneur comprend une plaque de base et un couvercle cylindrique de dimensions correspondantes fait dans un verre borosilicate et en ce que la première couche métallique déposée est en titane, la seconde couche en or et que l'élément de soudure est fait d'un alliage de SnInAg.

8. Dispositif médical implantable, **caractérisé par le fait que** les composants électroniques dudit dispositif sont encapsulés dans un conteneur en verre comprenant deux éléments qui sont soudés ensemble selon un procédé selon la revendication 1 de manière à former un conteneur étanche.

9. Senseur de pression implantable, **caractérisé par le fait qu'**il comprend un transducteur de pression soudé sur une plaque de base en verre borosilicate présentant une ouverture sur une partie de sa surface et en ce qu'un couvercle en verre borosilicate est soudé sur la plaque de base à basse température selon un procédé revendiqué dans l'une des revendications 1 à 5 de manière à obtenir une liaison étanche entre le couvercle et la plaque de base.

10. Senseur de pression implantable selon la revendication 9, **caractérisé par le fait que** le transducteur de pression est lié à un support en verre borosilicate et en ce que le support est soudé selon le procédé de la revendication 1 à la plaque de base d'un conteneur en verre borosilicate comprenant une plaque de base et un couvercle correspondant.

11. Conteneur en verre pour encapsuler des composants électroniques destinés à être implanté dans un corps humain ou animal, **caractérisé par le fait qu'**il comprend un premier élément et un second élément formant un couvercle, les deux éléments étant soudés ensemble selon le procédé de la revendication 1.
